(19) Europäisches Patentamt
European Patent Office
Office européen des brevets

(11) Publication number: **0 190 003 B1**

(12) **EUROPEAN PATENT SPECIFICATION**

(45) Date of publication of patent specification: **11.09.91**

(51) Int. Cl.[5]: **G03C 1/815**, C08F 20/36, //C07D249/20

(21) Application number: **86300416.4**

(22) Date of filing: **22.01.86**

The file contains technical information submitted after the application was filed and not included in this specification

# (54) UV-Absorbing polymers and photographic materials containing them.

(30) Priority: **22.01.85 GB 8501563**

(43) Date of publication of application:
**06.08.86 Bulletin 86/32**

(45) Publication of the grant of the patent:
**11.09.91 Bulletin 91/37**

(84) Designated Contracting States:
**DE FR GB**

(56) References cited:
**EP-A- 0 057 160**
**EP-A- 0 131 468**
**GB-A- 1 346 764**
**US-A- 3 813 255**

(73) Proprietor: **EASTMAN KODAK COMPANY (a New Jersey corporation)**
**343 State Street**
**Rochester New York 14650(US)**
(84) Designated Contracting States:
**DE FR**

Proprietor: **Kodak Limited**
**Kodak House Station Road**
**Hemel Hempstead Hertfordshire HP1 1JU(GB)**
(84) Designated Contracting States:
**GB**

(72) Inventor: **Schofield, Edward**
**111 Lynton Road**
**Chesham, Bucks., HP5 2BP(GB)**
Inventor: **Wagner, Hans Max**
**4 Dovercourt Gardens**
**Stanmore, Middx., HA7 4SH(GB)**
Inventor: **Daw, Christine Carol**
**40 Pheasants Way**
**Rickmansworth, Herts., WD3 2HA(GB)**

(74) Representative: **Baron, Paul Alexander Clifford et al**
**Kodak Limited Patent Department Headstone Drive**
**Harrow Middlesex HA1 4TY(GB)**

EP 0 190 003 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to UV-absorbing polymeric latices and their use in photographic photosensitive materials.

Photographic photosensitive materials, particularly colour materials often contain a layer comprising gelatin and a UV-absorber. Frequently this is an overlayer positioned farther from the support than any of the other layers of the material.

A UV-absorbing compound which has been used for this purpose has the formula:

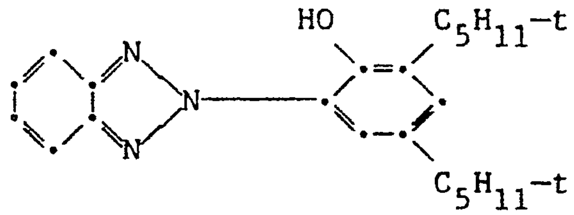

(I)

It has been observed that this compound is liable to crystallise out which results in migration of the compound to the surface causing an undesirable blooming effect.

British Patent Specification 1,346,764 describes the use of a UV-absorbing copolymer latex dispersed in a gelatin solution and coated to form a UV-absorbing layer. One of the polymers specified therein has repeating units of the formula:

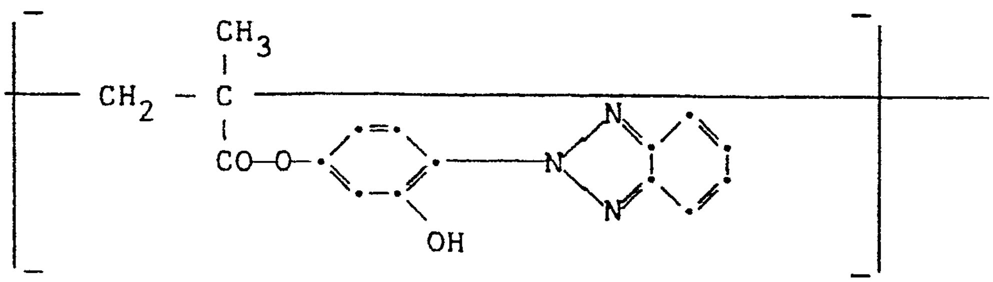

This polymer has spectral absorption characteristics that allow some unwanted UV radiation to be transmitted.

The present invention provides polymeric UV-absorbers which are free from the blooming referred to above. The present polymer comprises the UV-absorbing moiety linked to the polymer backbone by an ether linkage and its absorption characteristics are improved over the prior polymer referred to above. Figure 1 shows a comparison of the absorption spectrum of monomeric UV-absorber 1 (see Example 3 below) and polymer 2(c) of the present invention (see Example 2) when coated at equal weights on transparent supports. It can be seen that the polymer 2(c) has a higher extinction coefficient and sharper cutting long wavelength edge. This can provide increased protection against UV while not absorbing as much blue. Indeed, a similar comparison, but coated on a support containing an optical brightener, showed that the polymeric UV-absorber did allow the desired fluorescence while the monomeric UV-absorber did not.

According to the present invention there is provided a UV-absorbing homo- or copolymer having repeating units of the formula:

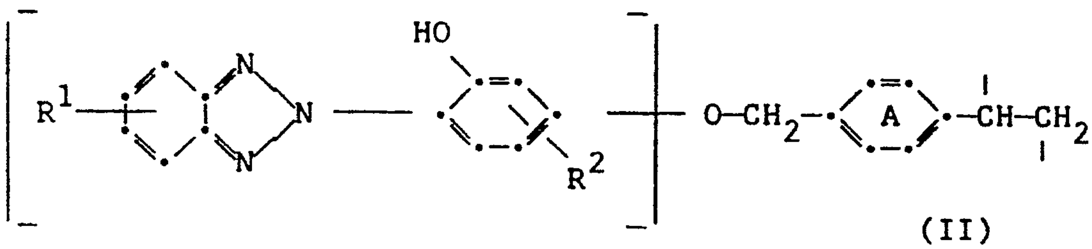

(II)

wherein

$R^1$ represents one or more halogen atoms or alkyl or alkoxy groups of 1-4 carbon atoms and

$R^2$ represents one or more halogen atoms or alkyl or alkoxy groups of 1-4 carbon atoms,

the benzene ring (A) being optionally substituted.

A preferred UV-absorbing polymer contains repeating units of the formula:

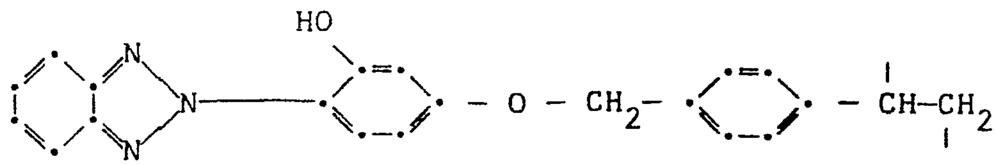

(III)

Copolymers according to the present invention may contain, in addition to repeating units of formula II, repeating units derived from styrene, methyl methacrylate, butyl methacrylate, vinyl acetate and/or 2-acrylamido-2-methylpropane sulphonic acid, and other similar monomers known in the art.

The present polymers are conveniently prepared as latices by methods of polymerisation, in themselves known, in the presence of a surfactant and an initiator. The surfactant can be ionic but is preferably non-ionic and those sold under the trade names OLIN 1OG SOLURIN PEN 95, DEREPHAT 154, TRITON X100 and TRITON X200E are suitable. The initiator may, for example, be sodium metabisulphate potassium persulphate or azo-bis-cyanovaleric acid.

The present latex polymers preferably have a particle size below 50 nm, especially 25 nm or below.

The present invention further provides a photosensitive photographic material comprising a support, at least one photographic silver halide emulsion layer and a gelatin layer having dispersed therein particles of a polymer or copolymer according to the present invention.

The advantages of using the latex UV-absorber compared to the prior art monomeric absorber without affecting developability or abrasion sensitivity are as follows:

1) Does not bloom
2) Can be coated in supercoat
3) Six layers instead of seven
4) Protection for whole pack
5) Better gloss
6) Little or no wet-haze
7) Higher Dmax and upper scale contrast in all layers (Reflection densitometry)
8) All aqueous coating
9) Equivalent or improved light-stability.

Since the UV-absorbing layers show minimum wet haze this allows, for example, wet prints to be assessed before drying. In addition the shape of the near UV-absorption curve is highly desirable leading to excellent protection from the deleterious effects of UV radiation.

The silver halide emulsions contained by the photographic materials of the present invention may contain negative-working or direct-positive silver halide emulsions. Illustrative useful emulsions are disclosed in Research Disclosure, Vol. 176, December 1978, Item 17643. The use of high aspect ratio tabular grain silver halide emulsions, as disclosed in Research Disclosure, Vol. 225, January 1983, Item 22534, is specifically contemplated. Research Disclosure and Product Licensing Index are publications of Kenneth Mason Publications Limited, Emsworth, Hampshire PO10 7DD, United Kingdom.

The layers of the photographic material can be coated on any conventional photographic support. Typical photographic supports include polymer film, wood fibre, e.g., paper, metallic sheet and foil, glass and ceramic supporting elements provided with one or more subbing layers to enhance the adhesive, antistatic, dimensional, abrasive, hardness, frictional, antihalation, and/or other properties of the support surfaces. Typical useful supports are further disclosed in Research Disclosure, Item 17643, cited above, Paragraph XVII.

The photographic elements can, of course, contain other conventional additives known in the art as described in Research Disclosure, Item 17643, cited above.

The present photographic materials may be multilayer colour materials forming images by reacting (coupling) a colour-developing agent (e.g. a primary aromatic amine) in its oxidised form with a dye-forming coupler. The dye-forming couplers can be incorporated in the photographic elements, as illustrated by Schneider et al, Die Chemie, Vol. 1944, p. 113, U.S. Patent 2,304,940, 2,269,158, 2,322,027, 2,376,679, 2,801,171, 3,748,141, 2,772,163, 2,835,579, 2,533,514, 2,353,754 and 3,409,435, and Chen Research Disclosure, Vol. 159, July 1977, Item 15930.

The dye-forming couplers upon coupling can release photographically useful fragments, such as development inhibitors or accelerators, bleach accelerators, developing agents, silver halide solvents, toners, hardeners, fogging agents, antifoggants, competing couplers, chemical or spectral sensitizers and desensitisers. Development inhibitor-releasing (DIR) couplers are illustrated by U.S. Patents 3,148,062, 3,227,554, 3,733,201, 3,617,291, 3,703,375, 3,615,506, 3,265,506, 3,620,745, 3,632,345, 3,869,291, 3,642,485, 3,770,436 and 3,808,945 and U.S. Patents 1,201,110 and 1,236,767. DIR compounds which do not form dye upon reaction with oxidised colour-developing agents can be employed, as illustrated by German OLS 2,529,350, 2,448,063, and 2,610,546 and U.S. Patents 3,928,041, 3,958,993, 3,961,959, 4,049,455 and 4,052,213. DIR compounds which oxidatively cleave can be employed, as illustrated by U.S. Patents 3,379,529, 3,043,690, 3,364,022, 3,297,445 and 3,287,129.

The photographic elements can incorporate coloured dye-forming couplers, such as those employed to form integral masks for negative colour images, as illustrated by U.S. Patents 2,449,966, 2,521,908, 3,034,892, 3,476,563, 3,519,429, 2,543,691, 3,028,238 and 3,061,432 and U.K. Patent 1,035,959 and/or competing couplers, as illustrated by U.S. Patents 3,876,428, 3,580,722, 2,998,314, 2,808,329, 2,742,832 and 2,689,793.

Combinations of UV absorbers are also useful. For example, the UV absorbers according to the invention can be used in combination with at least one of the UV absorbers known in the photographic art, such as one or more of the UV absorbers described in, for example, European Patent Application 57160; U.S. 4,540,656; U.S. 4,431,726; U.S. 3,253,921; U.S. 4,464,462; and U.S. 4,455,368.

The UV absorber according to the invention can be in any location in a photographic element which enables the desired characteristics, including desired UV absorbing properties. For example, the UV absorber can be in any layer or combination of layers of the photographic element; however, the UV absorber is particularly advantageous in an overcoat layer and/or in one or more of the layers of the photographic element farthest from the support.

The following Examples are included for a better understanding of the invention.

Example 1

2-[2-hydroxy-4-(4-vinyl)benzyloxyphenyl]benzotriazole (MonomerI)

A solution of sodium nitrite (55.2 g) in water (100 $cm^3$) was added dropwise to a stirred suspension of o-nitroaniline (110.4 g) in concentrated hydrochloric acid (640 $cm^3$) keeping the temperature below 0° C. The mixture was stirred for a further 2 hours at 0° C, filtered and added dropwise to a well stirred mixture of resorcinol (132 g), concentrated hydrochloric acid (120 $cm^3$), ice (4 kg) and water (10 $dm^3$).

The orange dye was filtered off, washed with water, slurried in water (5 $dm^3$) and nitrogen bubbled through the mixture. Fresh zinc powder (140 g) was added followed by sodium hydroxide (440 g) in water (1 $dm^3$) and the mixture heated on a steam bath for 2 hours until the red colour was discharged.

After allowing to settle the supernatent solution was poured under nitrogen onto ice (3 kg) and hydrochloric acid (2M) was poured into the stirred mixture until the product began to precipitate.

The crude product was filtered, dried, and recrystallised from ethanol. (Yield 105 g).

80 g of this product was refluxed with potassium carbonate (24.6 g), sodium iodide (5.3 g) and topanol OC (0.3 g) in butanone (250 $cm^3$). Chloromethylstyrene (59.8 g) in butanone (175 $cm^3$) was added in 4 portions over 4 hours. $K_2CO_3$ (12.3 g) and NaI (1.8 g) was also added before each subsequent addition of chloromethylstyrene.

The mixture was further refluxed for 24 hours, cooled to room temperature, the inorganic material filtered off and the mixture evaporated to dryness. The product was recrystallised from ethanol. (yield 51.5 g).

Calc. for $C_{12}H_9N_3O_2$ :
C, 63.45; H,3.96; N, 18.50; M, 227.

Found :
C, 63.29; H,3.97; N, 18.6; $M^+$ 227.

Example 2 Typical latex preparation

(a) Sodium lauryl sulphate (SLS) (1 g) dissolved in water (150 $cm^3$) was heated to 80°C with stirring under nitrogen. Sodium metabisulphite (0.05 g) and potassium persulphate (0.4 g) were added and the reaction mixture stirred at 80° C for 15 minutes. 2-acrylamido-2-methyl-propane sulphonic acid (0.5 g) as a 10% aqueous solution, pH 5 was added followed by a solution of monomer I (4 g) in dioxan (20 $cm^3$) and vinyl acetate (10 $cm^3$). Sodium metabisulphite (0.1 g) in water (10 $cm^3$) was added dropwise over 0.5 hour and the reaction mixture heated at 80°C for further 1.5 hours. The latex was cooled, filtered, purified by ulta-filtration and the volume reduced to 50 $cm^3$.
Solids 9.1%. UV absorption at λ max: 0.96 when diluted 1:5000 with $H_2O$. Particle size: ca 25 nm.
(b)-(e) Further samples of latex were prepared by analogous means according to the details given in Table 1 below in which BMA = butylmethacrylate and VA = vinyl acetate and AMS = 2-acrylamido-2-methylpropane sulphonic acid. Properties of the latices are given in Table 2.

TABLE 1

Reaction conditions for the preparation of latex samples

| Example No. | SLS (g) | $K_2S_2O_8$ (g) | $NA_2S_2O_5$ (g) | (I) (g) | Comonomer ($cm^3$) | (AMS) (g) | $H_2O$ ($cm^3$) | THF ($cm^3$) |
|---|---|---|---|---|---|---|---|---|
| 2(a) | 1.0 | 0.4 | 0.15 | 0.4 | 10 (VA) | 0.5 | 150 | 4** |
| 2(b) | 0.3 | 0.2 | 0.3 | 1.5 | 7 (BMA) | 0.5 | 60 | 10 |
| 2(c) | 1.0 | 0.2 | 0.3 | 4.0 | 8 (VA) | 0.5 | 100 | 15 |
| 2(d) | 0.5 | 0.4 | 0.6 | 3.0 | 20 (VA) | 1.0 | 100 | 20 |
| 2(e) | 1.0 | 0.2 | 0.3 | 4.0 | 8 (BMA) | 0.5 | 100 | 15 |

TABLE 2

Data for Latex samples

| Example No. | Final Volume ($cm^3$) | Solids % | X:Y* |
|---|---|---|---|
| 2(a) | 105 | 2.5 | 1:1.6 |
| 2(b) | 100 | 4.3 | 1:3.8 |
| 2(c) | 150 | 2.8 | 1:0.68 |
| 2(d) | 140 | 2.7 | 1:1.7 |
| 2(e) | 130 | 4.6 | 1:2.0 |

*UV absorber : other monomers in latex (wt/wt as determined by UV spectroscopy)

**Dioxan instead of THF

## Example 3

Coatings were prepared as shown below, with the different UV absorbing latices incorporated in a super-coat (Table 3) numbers in parentheses indicate laydown in mg/dm². Control coatings were prepared where (i) layer 07 contained no UV absorber and (ii) UV absorber 1 (7.9) was incorporated in layer 04 and layer 07 was omitted.

| | | | |
|---|---|---|---|
| 07 | GEL (64.6) | UV absorbing polymer | |
| 06 | GEL (10.5) | | |
| 05 | GEL (20.5) | Coupler 1<br>Scavenger 1<br>Red Sensitised Silver Halide Emulsion | (6.9)<br>(0.10)<br>(2.5) |
| 04 | GEL (10.4) | Scavenger 1 | (0.54) |
| 03 | GEL (18.3) | Coupler 2<br>Scavenger 1<br>Scavenger 2<br>Green Sensitised Silver Halide Emulsion | (5.4)<br>(0.54)<br>(2.2)<br>(4.7) |
| 02 | GEL (10.4) | Scavenger 1 | (0.54) |
| 01 | GEL (7.3) | Coupler 3<br>Blue Sensitive Silver Halide Emulsion | (10.2)<br>(4.0) |

////////Poly(ethyleneterephthalate) base //////////////

The coatings were exposed through a split wedge with Wratten 70, 99 and 98 Filters, to give cyan, magenta and yellow exposures respectively, then processed as described below. Light (12 weeks 5.4k lx SANS) and dark (6 weeks 75% RH, 49°C) stability data for starting densitites of 1.0 are shown in Table 3. The compounds used in the above coatings were:

Coupler 1

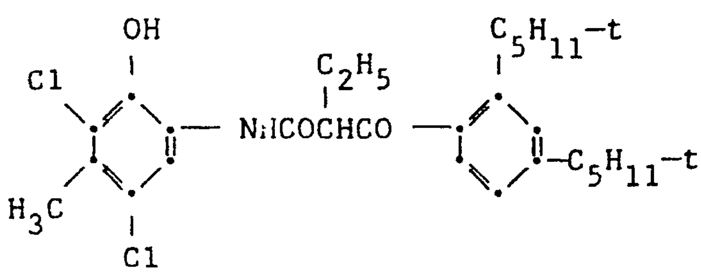

Coupler 2

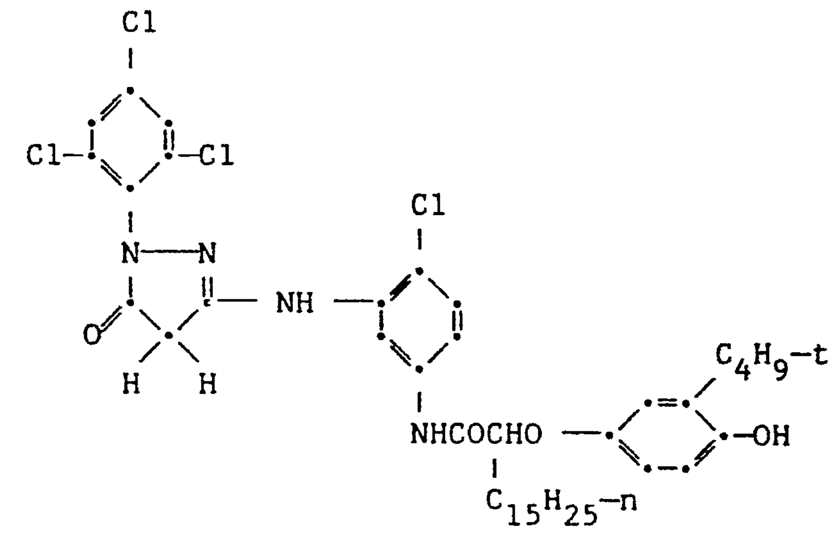

Coupler 3

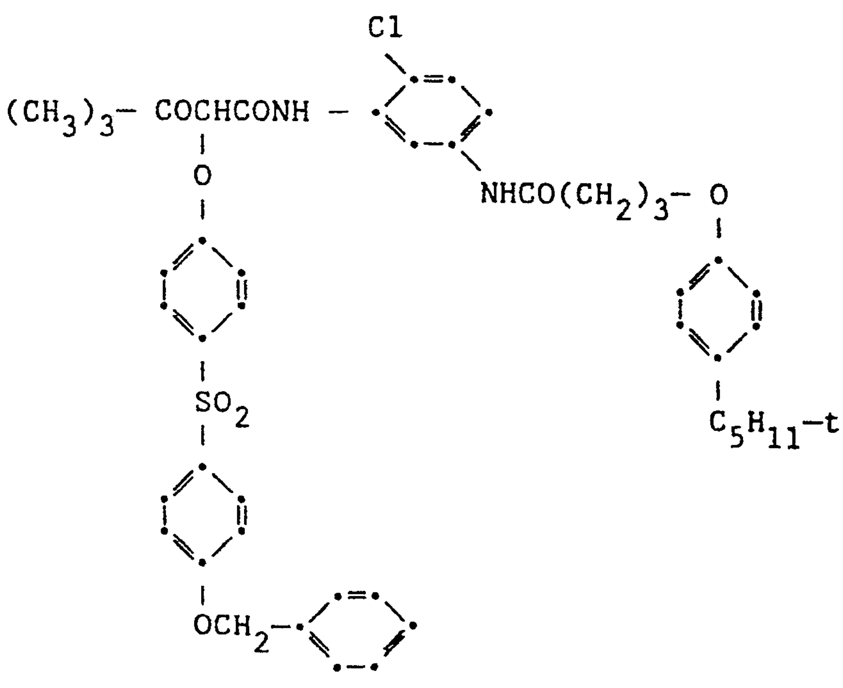

Scavenger 1

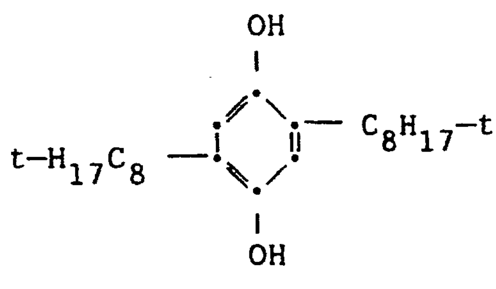

UV Absorber 1

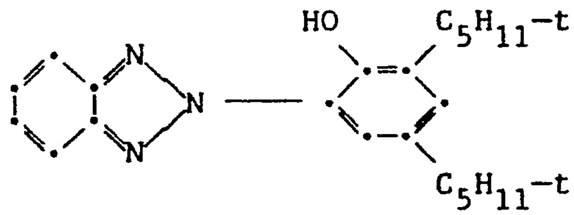

Scavenger 2

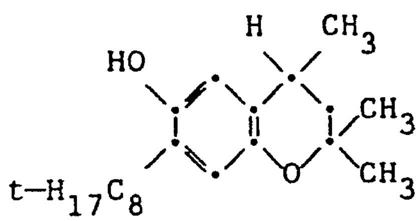

The processing was as follows:

Developer 3.5 minutes

Bleach-Fix 1.5 minutes

Wash 1.5 minutes

DEVELOPER

| | |
|---|---|
| Benzyl alcohol | 15.1 $cm^3/dm^3$ |
| Ethylene glycol | 21.1 $cm^3$ |
| Surfactant 10G* | 0.2 $cm^3$ |
| Phorwite (liquid)** | 2.5 $cm^3$ |
| Hydroxylamine sulphate | 3.86 g |
| 1-hydroxyethane-1,1-diphosphonic acid (as 10% solution) | 1.42 $cm^3$ |
| $Li_2SO_4$ | 2.74 g |
| $K^2SO_3$ (45%) | 3.24 $cm^3$ |
| 4-Amino-3-methyl-(N-ethyl-N-(2-methane-sulphonamidoethyl)-aniline sesquisulphate | 4.85 g |
| $K_2CO_3$ | 32 g |
| pH (27°C) | 10.08 |

*Surfactant 20G is an Olin Corp. Trade Mark

**Phorwite is a Bayer AG Trade Mark.

BLEACH-FIX

| | |
|---|---|
| $(NH_4)_2S_2O_3$ | 104 $g/dm^3$ |
| $NaHSO_3$ | 13 g |
| Ammonium ferric EDTA (.18M) | 65.6 g |
| EDTA | 6.56 g |
| $NH_4OH$ (28%) | 27.9 $cm^3$ |
| pH (27°C) | 6.8 |

TABLE 3

| Example | Laydown (mg dm$^{-2}$) | Absorbance at λ max | LIGHT STABILITY %ΔD* | | | DARK STABILITY ΔD | | |
|---|---|---|---|---|---|---|---|---|
| | | | CYAN | MAGENTA | YELLOW | CYAN | MAGENTA | YELLOW |
| 2(a) | 12.2 | 2.68 | 4.0 | 10.5 | 14.5 | −.020 | −.025 | −.025 |
| 2(b) | 20.9 | 2.46 | 4.0 | 11.0 | 14.0 | −.025 | −.030 | −.025 |
| 2(c) | 8.7 | 2.68 | 4.0 | 13.5 | 16.5 | −.025 | −.035 | −.025 |
| 2(d) | 13.1 | 2.72 | 4.5 | 12.0 | 16.0 | −.010 | −.030 | −.04 |
| 2(e) | 14.8 | 2.64 | 4.0 | 12.0 | 16.0 | −.015 | −.025 | −.025 |
| Control (i) | – | – | 4.5 | 32.5 | 28.0 | 0 | −.020 | −.040 |
| Control (ii) | 7.9 | 2.36 | 4.5 | 15.5 | 25.0 | −.045 | −.035 | −.010 |

* % loss in dye density calculated for $D_o = 1.0$ using the formula $[e^{2.303D}-1] = e^{aE}[e^{2.303\ D_o}-1]$ where

$D_o$ = initial density and D = density after fading,
E is the exposure and a is a constant.

## Example 4

Coatings were made having the following structure, the numbers in parentheses being the laydown in mg/dm².

| | | | | |
|---|---|---|---|---|
| 6: | GEL | (10.2) | UV-absorbing latex | (2.7) |
| 5: | GEL | (8.6) | Coupler 1 | (6.9) |
| | | | Scavenger 1 | (0.10) |
| | | | Red Sensitised Silver Halide Emulsion | (2.5) |
| 4: | GEL | (10.9) | Scavenger 1 | (0.54) |
| 3: | GEL | (13.9) | Coupler 2 | (5.4) |
| | | | Scavenger 1 | (0.54) |
| | | | Scavenger 2 | (2.2) |
| | | | Green Sensitised Silver Halide Emulsion | (4.7) |
| 2: | GEL | (9.2) | Scavenger 1 | (0.54) |
| 1: | GEL | (12.7) | Coupler 3 | (10.2) |
| | | | Blue Sensitive Silver Halide Emulsion | (4.0) |

////////Poly(ethyleneterephthalate)base/////////////////

Coating (a) contained a latex described in British Specification 1,346,764 comprising units of the formula:

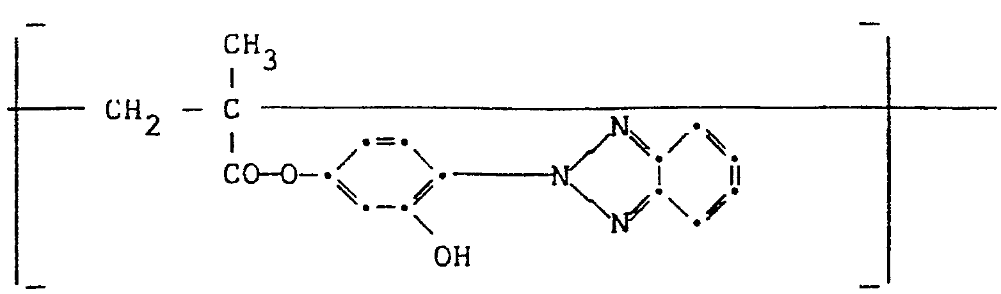

while coating (b) contained the latex of Example 2(a).

The absorption spectra of the resulting coatings are shown in Fig. 2 of the accompanying drawings. It can be seen that the present UV-absorbing latex has superior absorption properties in the critical 350-400 nm region. Below 350 nm, the film base itself starts absorbing UV radiation.

Example 5

In order to compare the smoothness of the UV-absorbing layer, coatings of the format described in Example 4 were made. Coating (a) contained 4.3 $mg/dm^2$ UV-absorber 1 in layer 6 while coating (b) contained 4.3 $mg/dm^2$ of a UV-absorbing polymer closely similar to 2(c) of Example 2. Both coatings also contained 4.3 $mg/dm^2$ UV-absorber 1 in layer 4. Surface gloss was measured on the Gardiner Glossmeter.

The results were as follows:

| Coating | Gloss value |
|---|---|
| a | 94.7 |
| b | 102.1 |

It can be seen that the UV-absorbing polymer imparts superior gloss in comparison to the conventional monomeric UV-absorber 1 when each is placed in the top coated layer. This also results in higher measured maximum reflection densities, particularly for the yellow image areas.

Example 6

Colour image transfer receiver materials were prepared according to the following format in order to compare the latex UV-absorber with a control dispersion containing a 3:1 by weight mixture of UV-absorbers 1 and 2. The latter requires a gelatin overcoat to prevent "blooming" and haziness due to crystallization of the UV-absorber mixtures. The number in parentheses is the laydown in $mg/dm^2$.

| | | |
|---|---|---|
| 4: | GEL (6.5) | |
| 3: | GEL (12.2) | UV-absorber (as in Table)<br>Formaldehyde (0.2) |
| 2: | GEL (30.7) | Cationic mordant (24.2)<br>Scavenger 2 (6.5)<br>Formaldehyde (0.5) |
| 1: | GEL (8.1) | |

//////////////Gel Subbed Baryta Paper Support//////////

Light-sensitive donor sheets were exposed, soaked in activator solution, and laminated with each of samples a and b for 10 minutes in the same manner as described in U.S. Patent 4,463,080 Example 3. Colour reflection densities in D-max image areas were determined as follows:

| Sample | UV-Absorber | Overcoat Layer 4 | D-max R | D-max G | D-max B |
|---|---|---|---|---|---|
| a | Control (8.1) | present | 2.51 | 2.30 | 2.10 |
| b | Latex 2(c) (8.6) | omitted | 2.65 | 2.52 | 2.43 |

The structures of Scavenger 2 and UV-Absorber 1 are given in Example 3 while other structures are as follows:

UV Absorber 2

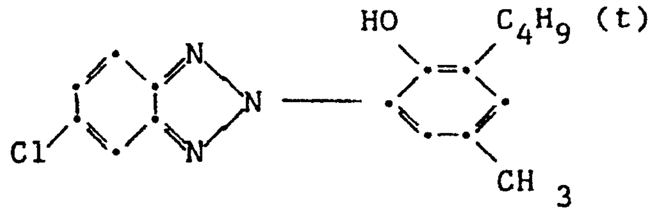

Cationic Mordant

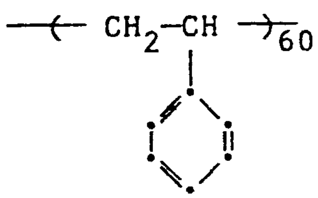

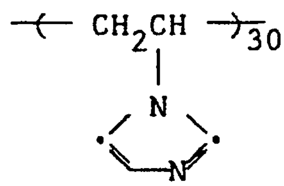

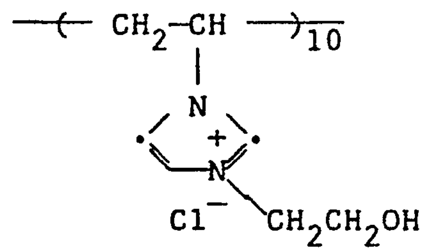

It can be seen that the latex UV-absorber of the invention allows omission of the gel overcoat resulting in desirable image dye density enhancement of all colours.

**Claims**

**1.** A UV-absorbing homo- or copolymer having repeating units of the formula:

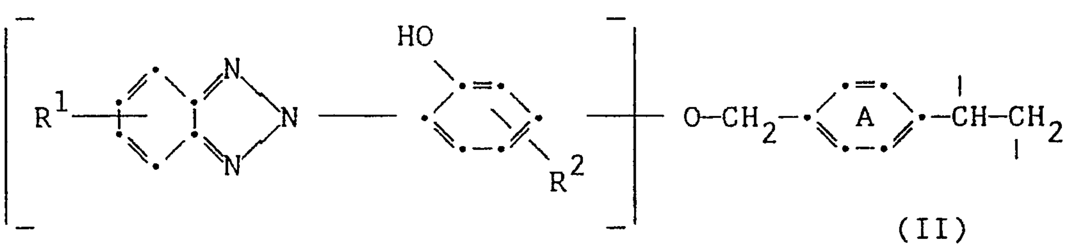

(II)

wherein

$R^1$ represents one or more halogen atoms or alkyl or alkoxy groups of 1-4 carbon atoms and

$R^2$ represents one or more halogen atoms or alkyl or alkoxy groups of 1-4 carbon atoms,

the benzene ring (A) being optionally further substituted.

**2.** A polymer which contains repeating units of the formula:

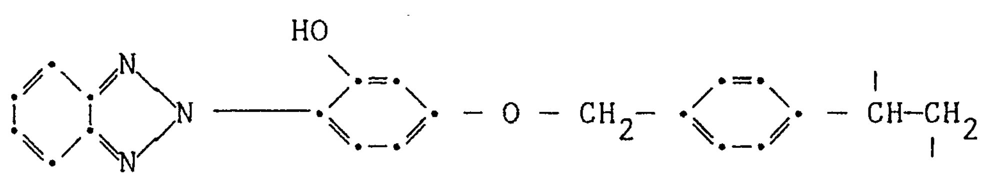

(III)

3. A polymer as claimed in claim 1 or 2 which further contains repeating units derived from styrene, methyl methacrylate, butyl methacrylate, vinyl acetate and/or 2-acrylamido-2-methylpropane sulphonic acid.

4. A polymer as claimed in any of claims 1-3 which is in the form of a latex having a particle size below 50 nm, preferably below 25 nm.

5. A photosensitive photographic material comprising a support, at least one photographic silver halide emulsion layer and a gelatin layer having dispersed therein particles of a polymer or copolymer according to claims 1-4.

6. A photographic material as claimed in claim 5 which is a multilayer colour material in which the polymer particles are present in at least the layer furthest from the support.

**Revendications**

1. Homopolymère ou copolymère absorbant le rayonnement ultraviolet comprenant des motifs de formule :

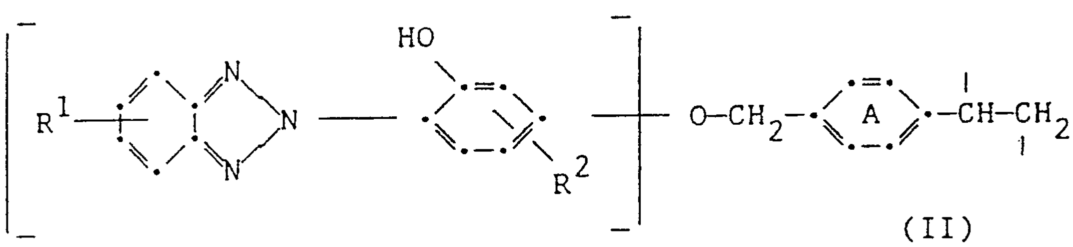

(II)

où

$R^1$ représente un ou plusieurs atomes d'halogène ou un groupe alkyle ou alkoxy de 1 à 4 atomes de carbone et,

$R^2$ représente un ou plusieurs atomes d'halogène ou des groupes alkyle ou alkoxy de 1 à 4 atomes de carbones,

le cycle benzenique (A) pouvant être de plus, substitué.

2. Polymère qui contient des motifs de formule :

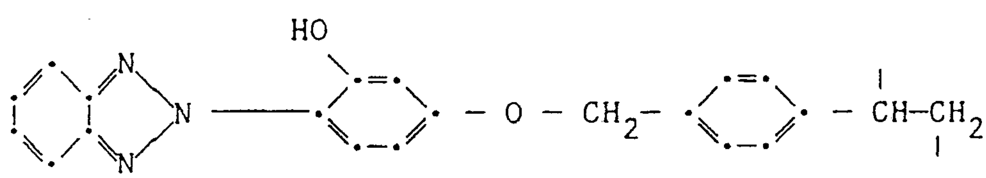

(III)

3. Polymère selon la revendication 1 ou 2 qui contient, de plus, des motifs dérivés du styrène, du méthacrylate de méthyle, du méthacrylate de butyle, de l'acétate de vinyle et/ou de l'acide 2-acrylamido-2-méthylpropane sulfonique.

4. Polymère selon l'une quelconque des revendications 1 à 3 qui est sous la forme de latex ayant une taille de particules inférieure à 50 nm, de préférence inférieure à 25 nm.

5. Produit photographique photosensible comprenant un support, au moins une couche d'émulsion photographique aux halogénures d'argent et une couche de gélatine dans laquelle sont dispersées les particules du polymère ou du copolymère selon les revendications 1 à 4.

6. Produit photographique selon la revendication 5 qui est un produit photographique multicouche en couleurs dans lequel les particules de polymère sont présentes au moins dans la couche la plus éloignée du support.

## Patentansprüche

1. UV-absorbierendes Homo- oder Copolymer mit wiederkehrenden Einheiten der Formel:

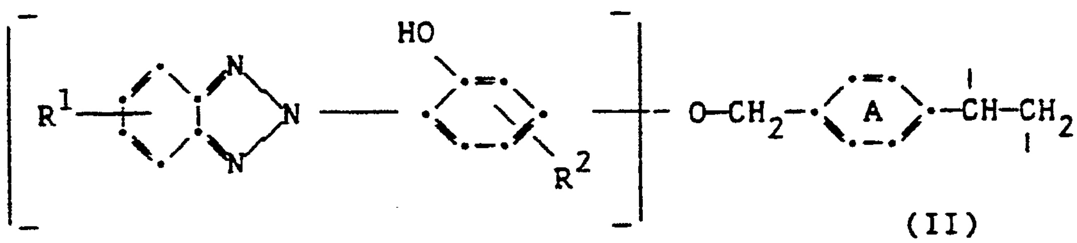

(II)

in der bedeuten:

$R^1$ ein oder mehrere Halogenatome oder Alkyl- oder Alkoxy-gruppen mit 1 - 4 Kohlenstoffatomen und

$R^2$ ein oder mehrere Halogenatome oder Alkyl- oder Alkoxy-gruppen mit 1 - 4 Kohlenstoffatomen,

wobei der Benzolring (A) gegebenenfalls weiter substituiert sein kann.

2. Polymer, das wiederkehrende Einheiten der Formel enthält:

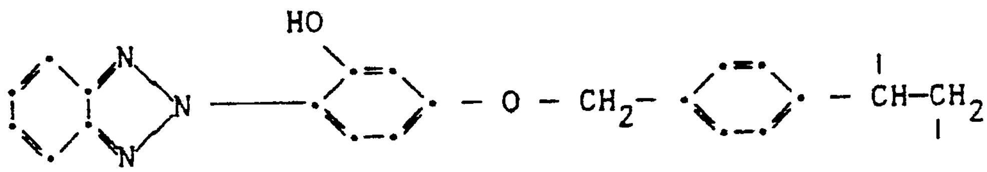

(III)

3. Polymer nach Anspruch 1 oder 2, das ferner wiederkehrende Einheiten aufweist, die sich von Styrol, Methylmethacrylat, Butylmethacrylat, Vinylacetat und/oder 2-Acrylamido-2-methylpropansulphonsäureableiten.

4. Polymer nach einem der Ansprüche 1 - 3, das in Form eines Latex mit einer Teilchengröße von unter 50 nm, vorzugsweise unter 25 nm vorliegt.

5. Photosensitives photographisches Material mit einem Träger, mindestens einer photographischen Silberhalogenidemulsionsschicht und einer Gelatineschicht, in der Teilchen eines Polymeren oder Copolymeren nach Ansprüchen 1 - 4 dispergiert vorliegen.

6. Photographisches Material nach Anspruch 5, bestehend aus einem mehrschichtigen farbphotographischen Material, in dem die Polymerteilchen in mindestens der Schicht vorliegen, die vom Träger am weitesten entfernt ist.

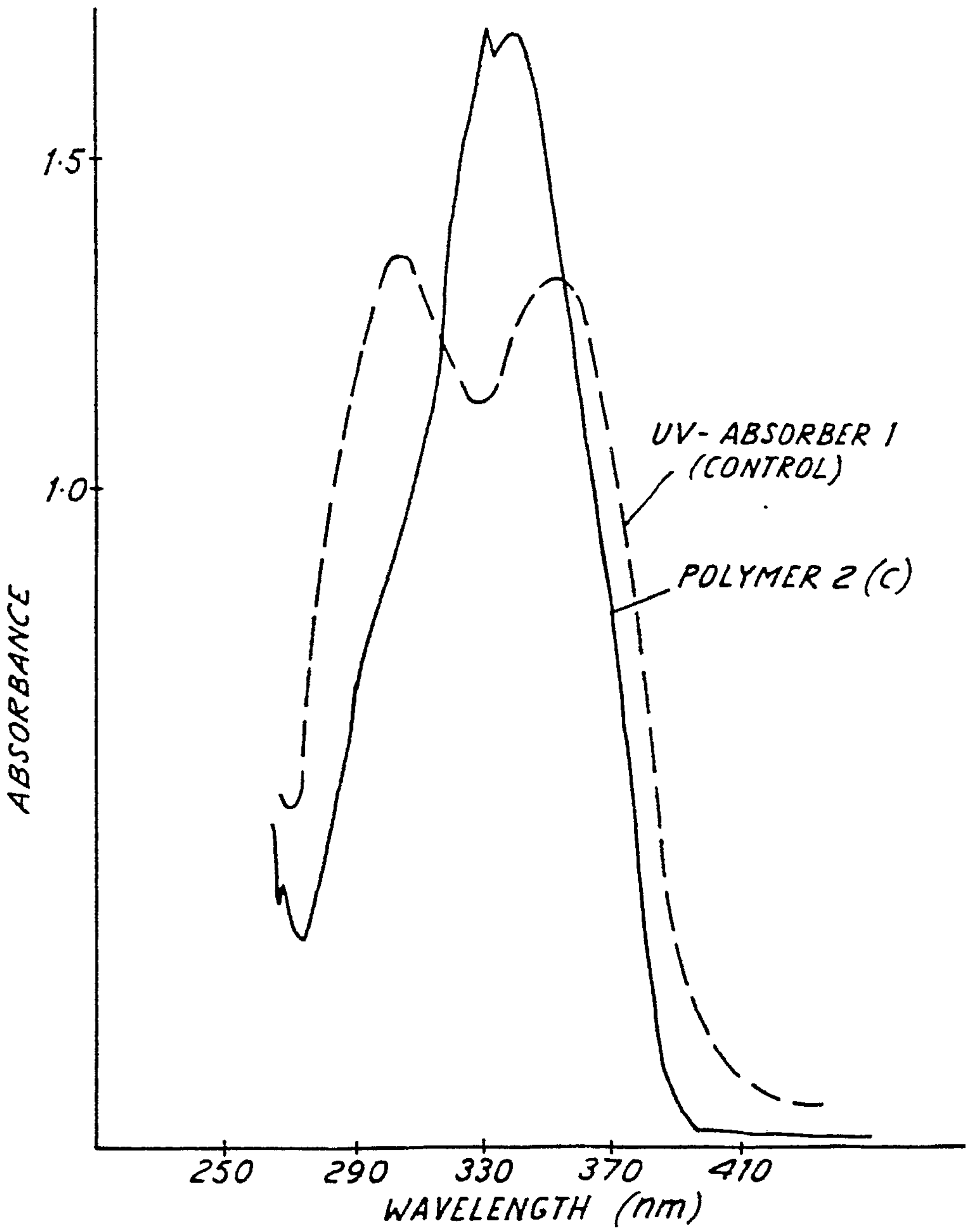
1·5
1·0
ABSORBANCE
UV- ABSORBER 1
(CONTROL)
POLYMER 2 (C)
250
290
330
370
410
WAVELENGTH (nm)

FIG. 1

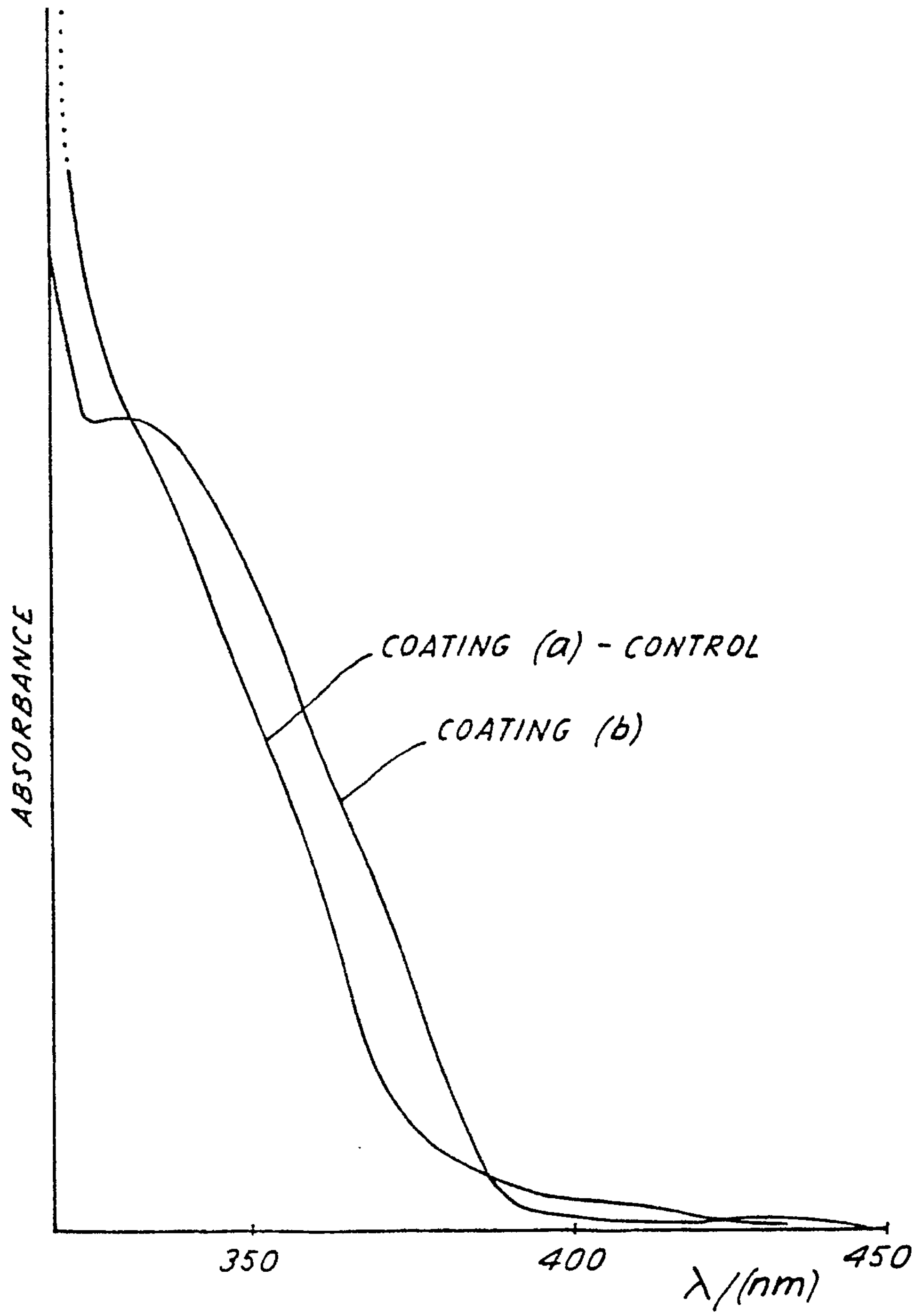


FIG. 2